# EUROPÄISCHE PATENTSCHRIFT

(11) **EP 0 446 867 B1**
(45) Veröffentlichungstag und Bekanntmachung des Hinweises auf die Patenterteilung: **17.07.1996**
(21) Anmeldenummer: 91103749.7
(22) Anmeldetag: 12.03.1991
(51) Int. Cl.: A61F 13/15

(54) **Wegwerfhöschen**
Disposable nappy
Couche culotte à jeter

(30) Priorität: 12.03.1990 JP 60768/90; 27.09.1990 JP 259834/90
(43) Veröffentlichungstag der Anmeldung: 18.09.1991
(73) Patentinhaber: UNI-CHARM CORPORATION, Kawanoe-shi Ehime-ken (JP)
(72) Erfinder: Igaue, Takamitsu, Kawanoe-shi, Ehime-ken (JP); Nomura, Hironori, Iyomishima-shi, Ehime-ken (JP); Shimakawa, Taiji, Kanonji-shi, Kagawa-ken (JP); Sasaki, Tohru, Kawanoe-shi, Ehime-ken (JP)
(74) Vertreter: Sperling, Rüdiger, Dipl.-Ing.

(56) Entgegenhaltungen:
- EP-A- 0 187 726
- EP-A- 0 214 636
- EP-A- 0 219 326
- EP-A- 0 251 332
- EP-A- 0 329 160
- GB-A- 2 161 059
- US-A- 4 747 846

## Beschreibung

Die Erfindung betrifft Wegwerfhöschen, insbesondere Windelhöschen für Babys, deren jeweilige Beinöffnungen jeweils mit doppelt ausgeführten elastisch dehnbaren Dichtelementen versehen sind (US-A- 4 747 846).

Es sind Wegwerfwindelhöschen für Babys bekannt, bei denen die Hüftöffnung und die beiden Beinöffnungen mit elastischen Elementen versehen sind. Mit diesen Windelhöschen bekleidete Kleinkinder sind jedoch im allgemeinen beim Herumlaufen und in ihren Bewegungen recht aktiv, so daß an den Beinöffnungen oft Exkremente austreten.

Demgemäß ist es Aufgabe der Erfindung, Wegwerfhöschen aufzuzeigen, die des weiteren Bündchen aufweisen, die von den Außenrändern der jeweiligen Beinöffnungen im Abstand angeordnete elastische Elemente enthalten, so daß das vorstehend genannte Problem beseitigt ist.

### BESCHREIBUNG DER ERFINDUNG

Gemäß der vorliegenden Erfindung wird die oben dargelegte Aufgabe durch Wegwerfhöschen mit einer Hüftöffnung mit einem ersten elastischen Element und zwei Beinöffnungen mit ringartigen zweiten elastischen Elementen gelöst, wobei zwei von den jeweiligen zweiten elastischen Elementen im Abstand nach innen angeordnete Bündchenelemente vorgesehen sind, die so ausgelegt sind, daß sie unter der Zugkraft von in ihnen enthaltenen dritten elastischen Elementen zur Haut des Benutzers hin angehoben werden.

Dabei sind bei aufgefaltetem Höschen bzw. Bekleidungsstück, dessen jeweils gegenüberliegende Seitenränder des vorderen und hinteren Teils noch nicht miteinander verbunden sind, die zweiten elastischen Elemente so positioniert, daß sie in einer Kurve nach innen verlaufen, während die dritten elastischen Elemente im wesentlichen parallel zur Längsachse des Höschens angeordnet sind, so daß nach dem Anlegen an den Körper des Benutzers von den zweiten elastischen Elementen gebildete elastisch dehnbare Linien teilweise elastisch dehnbare Linien überschneiden, die von den dritten elastischen Elementen gebildet werden, um so die Bündchenelemente zur Haut des Benutzers hin zu spannen.

Vorzugsweise umfassen eine innere Lage, die eine Innenfläche des Höschens bildet, eine elastisch dehnbare, wasserdurchlässige Schicht, eine äußere Lage, die eine Außenfläche des Höschens bildet, eine elastisch dehnbare, wasserundurchlässige Schicht, und die Bündchenelemente feuchtigkeitsdurchlässige Schichten.

Bei den erfindungsgemäßen Höschen mit dem vorstehend beschriebenen Aufbau sind die in gewissem Abstand innerhalb der zweiten elastischen Elemente angeordneten Bündchenelemente so eingerichtet, daß sie durch die Kontraktionskraft der in ihnen enthaltenen dritten elastischen Elemente an die Haut des Benutzers gehoben werden und die jeweiligen Beinöffnungen so mit inneren und äußeren Dichtelementen versehen sind. Dadurch wird möglicherweise auftretendes Austreten von Exkrementen am Umfang der Beinöffnungen wirksam verhindert, auch wenn der Benutzer aktiv läuft und sich bewegt.

Nach dem Anlegen an den Körper des Benutzers überschneiden die von den ersten elastischen Elementen gebildeten elastisch dehnbaren Linien teilweise die von den dritten elastischen Elementen gebildeten elastisch dehnbaren Linien, um so die Bündchenelemente gegen die Haut des Benutzers vorzuspannen. Dieses Merkmal hat die Wirkung, daß das Umlegen der Bündchenelemente nach außen verhindert wird, so daß die Bündchenelemente als wirksame Sperren für Exkremente dienen. Mit anderen Worten halten die Bündchenelemente die Exkremente an ihrer Innenseite. Entsprechend ist das Verhindern eines Exkrementeaustritts an den Beinöffnungen weiter verbessert.

Die innere bzw. äußere Schicht, die die Innen- bzw. Außenfläche des Höschens bildet, enthält elastisch dehnbares Material, so daß die innere und äußere Schicht dehnbar und zusammenziehbar ausgelegt sind, um sich auf die Bewegungen des Benutzers einzustellen. Dieses Merkmal bietet Passgenauigkeit und Tragekomfort für den Träger des Höschens.

### KURZE BESCHREIBUNG DER ZEICHNUNGEN

Eine Ausführungsform des erfindungsgemäß aufgebauten Höschens wird von den beigefügten Zeichnungen gezeigt, wobei
- Fig. 1: eine perspektivische Darstellung dieser Ausführungsform,
- Fig. 2: eine abgewickelte perspektivische Darstellung der Ausführungsform mit voneinander getrenntem Vorder- und Hinterteil,
- Fig. 3: eine auseinandergezogene Darstellung der Ausführungsform,
- Fig. 4: eine Schnittdarstellung entlang der Linie IV - IV in Fig. 2,
- Fig. 5: eine Seitenansicht der Ausführungsform, und
- Fig. 6: eine perspektivische Darstellung einer weiteren Ausführungsform der äußeren Schicht
zeigen.

### BEVORZUGTE AUSFÜHRUNGSFORM DER ERFINDUNG

Die vorliegende Erfindung wird anhand von Ausführungsformen unter Bezug auf die beiliegenden Zeichnungen beschrieben.

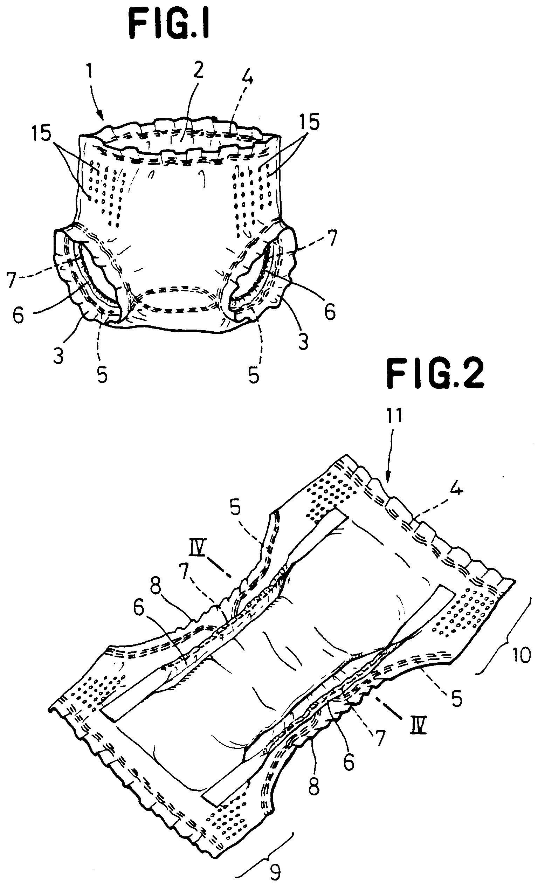

Wie in Fig. 1 und 2 gezeigt, hat das Höschen 1 eine Hüftöffnung 2 und zwei Beinöffnungen 3, die entlang den ihren Rändern benachbarten Bereichen mit in diesen befestigten, zugehörigen elastischen Elementen 4, 5 versehen sind. Weiter enthalten die Höschen 1 Bündchenelemente 6, die entlang den jeweiligen Beinöffnungen 3 mit Abstand zu den jeweiligen elastischen Elementen 5 innerhalb dieser angeordnet sind. Von den Bündchenelementen 6 ist jeweils ein elastisches Element 7 umhüllend eingeschlossen. Fig. 2 zeigt einen entfalteten Schichtkörper 11. Vorder- und Hinterteil 9, 10 des Schichtkörpers 11 können an ihren gegenüberliegenden Seitenrändern mit Ausnahme von die Beinöffnungen bildenden Abschnitten 8 miteinander verBündchenen werden, um so das hosenartige Kleidungsstück bzw. Höschen zu bilden.

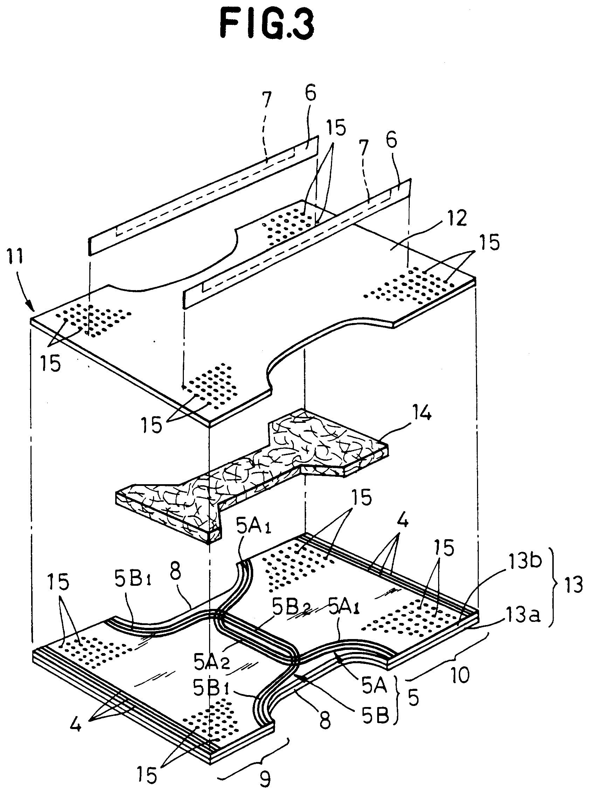

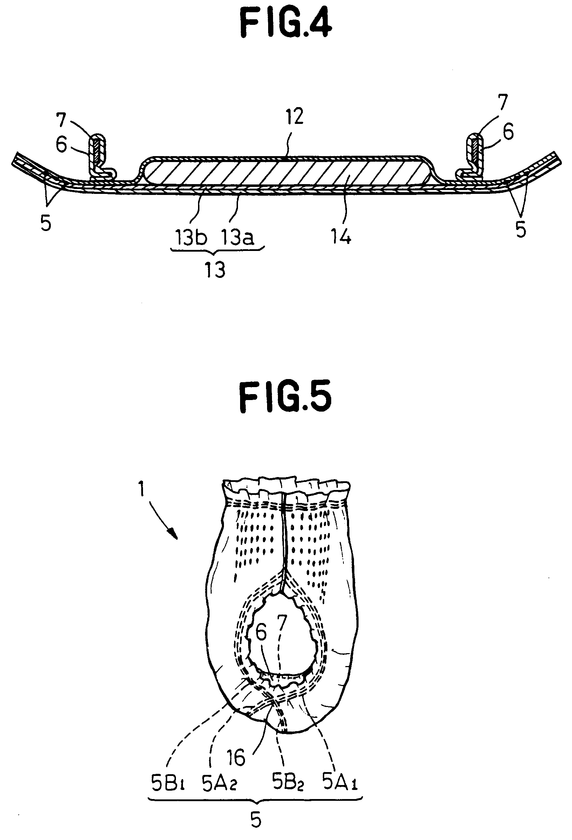

Wie in Fig. 3 und 4 dargestellt, umfaßt der Schichtkörper 11 eine innere Lage 12 aus elastischem, wasserdurchlässigen Faservliesstoff, die eine Innenfläche des Höschens bildet, eine äußere Lage 13 aus einer elastischen, wasserundurchlässigen Elastomerfolie 13b, wie z.B. Kunststoff- oder Gummifolie, die auf die Innenfläche eines Faservliesstoffes 13a, der im wesentlichen der inneren Lage 12 aus Faservliesstoff ähnlich ist, aufgelegt ist, die die Außenfläche des Höschens 1 bildet, und einem matten- oder schichtartigen, sanduhrförmigen Absorptionskern 14, der aus einer Mischung von Fasermasse, thermoplastischen Fasern und wasserunlöslichem, hoch wasserabsorptivem Polymerpulver geformt ist.

Der elastische, wasserdurchlässige Faservliesstoff kann vorzugsweise durch Kardieren von warmgekräuselten Fasern mit einem Grundgewicht von 25 bis 45g/m² und einer Feinheit von 0,5 bis 3 d zu einem Faserflor und anschließender Wärmebehandlung des Faserflors zur Erzielung einer dünnen Schichtform erhalten werden.

Die elastische Festigkeit (Dehnfestigkeit) der Elastomerfolie 13b ist vorzugsweise höher als die der Faservliesstoffe 12, 13a. Diese Bestandteile können jedoch auch eine im wesentlichen gleiche Festigkeit haben.

Ein vom jeweils die innere Lage 12 und die äußere Lage 13 enthaltenden Vorder- und Hinterteil 9, 10 begrenzter Schrittbereich ist entlang den gegenüberliegenden Seitenrändern mit in einer Kurve nach innen verlaufenden Ausnehmungen, die den jeweiligen vorstehend genannten Abschnitten 8, die die Beinöffnungen bilden, entsprechen, ausgebildet. Die die Hüftöffnung bildenden Abschnitte, die sich entlang den Längsenden des Vorder- und Hinterteils 9, 10 erstrecken, und die die jeweiligen Beinöffnungen bildenden Abschnitte 8 sind nahe an ihren Rändern mit den jeweiligen elastischen Elementen 4, 5 versehen, die zwischen der inneren Lage 12 und der Elastomerfolie 13b eingelegt sind. Die elastischen Elemente 4 für die Hüftöffnung sind mit Heißschmelzkleber in gedehntem Zustand mit der inneren Lage 12 und/oder der Elastomerfolie 13b parallel zu den Längsenden des Vorder- und Hinterteils 9, 10 verbunden. Die elastischen Elemente 5 für die Beinöffnungen umfassen ein erstes Element 5A und ein zweites Element 5B. Das erste Element 5A und das zweite Element 5B überkreuzen einander an nahe an den gegenüberliegenden Enden der jeweiligen Elemente 5A, 5B gelegenen Punkten. Abschnitte 5A₁, 5B₁, die von den jeweiligen Kreuzungspunkten nach außen verlaufen, sind ebenfalls mit Heißschmelzkleber in gedehntem Zustand mit der Innenfläche der inneren Lage 12 und/oder der Elastomerfolie 13b entlang den Rändern der jeweiligen, die Beinöffnungen bildenden Abschnitte 8 verbunden. Zwischen den beiden Kreuzungspunkten liegende Mittelabschnitte 5A₂, 5B₂ sind weder mit diesen Lagen noch mit dem Kern 14 verbunden und lediglich an der Unterseite des Kerns 14 in dessen mittlerem Bereich angeordnet.

Die obere Fläche des Kerns 14 ist an der inneren Lage 12 mit Heißschmelzklebepunkten befestigt. Dadurch kann nicht nur die Bewegung des Kerns 14 sehr gering gehalten werden, sondern es wird auch vermieden, daß die Absorption von flüssigen Ausscheidungen durch die innere Lage 12 hindurch in den Kern 14 mehr oder weniger behindert wird, wenn die innere Lage 12 von der oberen Fläche des Kerns 14 abgehoben ist. Des weiteren wird auch die Beeinträchtigung der elastischen Dehnung und Kontraktion der inneren und äußeren Lage 12, 13 dadurch möglichst gering gehalten.

An der oberen Fläche der inneren Lage 12 sind die Bündchenelemente 6 von den Endabschnitten 5A₁, 5B₁ der elastischen Elemente 5 im Abstand nach innen im wesentlichen parallel zur Längsachse des Schichtkörpers 11 angeordnet. Die Bündchenelemente 6 umfassen jeweils eine Lage eines feuchtigkeitsdurchlässigen, aber wasserundurchlässigen Faservliesstoffes, der zu einem Schlauch gefaltet ist. In eine Seite dieses Schlauchs ist das elastische Element 7 eingeschlagen und mit dieser in gedehntem Zustand mit Heißschmelzkleber verbunden, und die andere Seite ist ebenfalls mit Heißschmelzkleber mit der oberen Fläche der inneren Lage 12 verbunden. Das so aufgebaute Bündchenelement ist nach außen umgelegt und an den gegenüberliegenden Längsenden mittels Heißschmelzkleber mit der oberen Fläche der inneren Lage 12 verbunden. Es ist jedoch ebenso möglich, daß das Bündchenelement 6 nach innen umgelegt ist und an den gegenüberliegenden Längsenden mittels Heißschmelzkleber mit der oberen Fläche der inneren Lage 12 verbunden wird. Unabhängig davon, auf welche Seite das Bündchenelement 6 umgelegt wurde, wird es durch die Kontraktionskraft des elastischen Elements 7 auch aufgerichtet, nachdem es mit seinen gegenüberliegenden Längsenden mit der oberen Fläche der inneren Lage 12 verbunden wurde. Auch ist es offensichtlich möglich, das Bündchenelement 6 mittels eines Schweißverfahrens mit der oberen Fläche der inneren Lage 12 zu verbinden.

Am Schichtkörper 11 sind die innere und äußere Lage 12, 13 in außerhalb der jeweiligen Bündchenelemente 6 liegenden Bereichen des Vorder- und Hinterteils 9, 10 (d.h. in Bereichen, in denen kein Kern 14 vorhanden ist) mit mehreren Belüftungsöffnungen 15 versehen. Die Belüftungsöffnungen 15 haben jeweils einen Durchmesser von 0,2 bis 2 mm und werden von einer Gruppe von Heiznadeln oder -stiften , die in bestimmten Abständen angeordnet sind, vorzugsweise so gebildet, daß die am inneren Umfang der Öffnungen freiliegenden Faservliesstoffe 12, 13a durch Stoff der Elastomerfolie 13b, die ebenfalls am inneren Umfang jeder Belüftungsöffnung freiliegt, miteinander verschweißt werden.

Die innere Lage 12 und die äußere Lage 13 können nach Wunsch an ihrem äußeren Umfang mittels Heißsiegeln, Ultraschallschweißen oder Heißschmelzkleben verbunden werden.

Der so aufgebaute Schichtkörper 11 wird entlang seiner quer zur Längsrichtung verlaufenden Mittellinie zusammengefaltet, worauf die jeweiligen Seitenränder des Vorder- und Hinterteils 9, 10 heißversiegelt oder ultraschallverschweißt werden, um das in Fig. 1 gezeigte Höschen 1 zu bilden. Dieses Verschweißen bzw. Versiegeln wird angewendet, um das zufällige Abreißen der jeweils gegenüberliegenden Seitenränder des Vorder- und Hinterteils 9, 10 während der Benutzung zu verhindern, jedoch beim Ausziehen des Höschens das leichte Abreißen zu ermöglichen. Eine derartige Verschweißung ist vorzugsweise mit Unterbrechungen entlang den jeweils gegenüberliegenden Seitenrändern des Vorder- und Hinterteils 9, 10 vorgesehen.

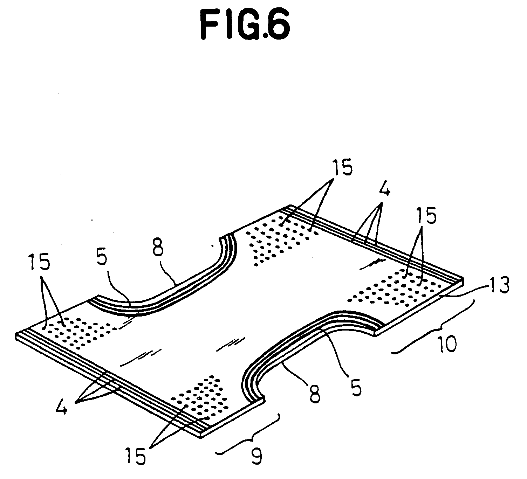

Fig. 5 zeigt das vollständig zusammengesetzte Höschen 1. Die von den Endabschnitten 5A₁, 5B₁ der elastischen Elemente 5 gebildeten elastisch dehnbaren Linien, die entlang den Beinöffnungen 3 verlaufen, überkreuzen die vom elastischen Element 7 im zugehörigen Bündchenelement 6, das innerhalb der Endabschnitte 5A₁, 5B₁ im Abstand angeordnet ist, gebildete elastisch dehnbare Linie und wirken dadurch in der Weise, daß die gegenüberliegenden Enden des Bündchenelements 6 nach innen vorgespannt werden, während ein Kreuzungspunkt 16 der Endabschnitte 5A₁, 5B₁ oder der Mittelabschnitte 5A₂, 5B₂ der elastischen Elemente 5 in der Weise wirkt, daß praktisch ein mittlerer Bereich des Bündchenelements 6 nach innen vorgespannt wird. Nach dem Anlegen an den Körper des Benutzers können die Höschen 1 bezüglich dem in Fig. 5 gezeigten Zustand in gewissem Ausmaß deformiert werden, aber die vorstehend genannte Vorspannungswirkung bleibt weiter bestehen, d.h., daß der die elastischen Elemente 5 enthaltende äußere Bereich das zugehörige Bündchenelement 6 nach innen vorspannt.

Die elastischen Elemente 5, 7 wirken offensichtlich unabhängig voneinander. Im einzelnen legen die elastischen Elemente 5 die Außenränder der jeweiligen Beinöffnung 3 an die Haut des Benutzers an und die elastischen Elemente 7 legen die jeweiligen Bündchenelemente 6 an die Haut des Benutzers an. Die vorstehend beschriebene Zusammenwirkung der von den elastischen Elementen 5 bzw. 7 gebildeten elastisch dehnbaren Linien erhöht die Zuverlässigkeit, mit der die Bündchenelemente 6 an die Haut des Benutzers angelegt werden. Entsprechend unterbinden die Bündchenelemente 6 in wirksamer Weise das Austreten von Exkrementen. Auch wenn eine gewisse Menge von Exkrementen über die Bündchenelemente 6 hinaus austritt, so kann diese von den Bereichen der jeweiligen Beinöffnungen, die die elastischen Elemente 5 enthalten, zurückgehalten werden.

Das Dazwischenlegen der Elastomerfolie 13b ist insofern vorteilhaft, als die Elastomerfolie das rasche Nachlassen der elastischen Festigkeit, insbesondere der Kontraktionsspannung, verhindert und eine gewünschte Kontraktionsspannung des Schichtkörpers sowie eine gewünschte Passform des Höschens am Körper des Benutzers aufrechterhält, auch wenn die Faserverschlingung oder Faserbindung des als Grundstoff für die innere Lage 12 und die äußere Lage 13 dienenden Faservliesstoffes beim Dehnen des Schichtkörpers gelöst wird.

Fig. 6 zeigt die perspektivische Darstellung einer weiteren Ausführungsform der äußeren Lage 13. Bei dieser Ausführungsform besteht die äußere Lage 13 aus einer einzelnen Schicht einer elastisch dehnbaren, wasserundurchlässigen Elastomerfolie wie z.B. einer Kunststoff- oder Gummifolie und die elastischen Elemente für die jeweiligen Beinöffnungen verlaufen nur um die jeweiligen Beine herum. Die übrigen Bestandteile entsprechen der vorhergehend beschriebenen Ausführungsform. Im Vergleich zu dieser wird bei dieser Ausführungsform in vorteilhafter Weise Material für die äußere Lage und die elastischen Elemente 5 eingespart und dadurch die entsprechende Reduzierung der Herstellungskosten des Höschens ermöglicht.

## Patentansprüche

1. Wegwerfhöschen, mit einer ein erstes elastisches Element (4) aufweisenden Hüftöffnung (2), mit einem Vorderteil (9) und einem Hinterteil (10) sowie jeweils daran angeordneten, einander gegenüberliegenden Seitenrändern, und mit zwei mit ringartigen, zweiten elastischen Elementen (5) versehenen Beinöffnungen, wobei jeweils ein in seinem freien Randbereich dritte elastische Elemente (7) aufweisendes Bündchenelement (6) im Abstand innerhalb der zweiten elastischen Elemente (5) angeordnet ist und von der Kontraktionskraft der dritten elastischen Elemente (7) zur Haut des Benutzers angehoben wird, **dadurch gekennzeichnet,** daß bei entfaltetem Höschen (1) mit noch nicht verbundenen gegenüberliegenden Seitenrändern eines Vorderteils (9) und eines Hinterteils (10) die zweiten elastischen Elemente (5) für die jeweiligen Beinöffnungen (3) so angeordnet sind, daß sie in einer Kurve nach innen verlaufen, während die dritten elastischen Elemente (7) für die Bündchenelemente (6) im wesentlichen parallel zur Längsachse des Höschens (1) positioniert sind, so daß nach dem Anlegen an den Körper des Benutzers von den zweiten elastischen Elementen (5) gebildete elastisch dehnbare Linien teilweise von den dritten elastischen Elementen (7) gebildete elastisch dehnbare Linien überschneiden.

2. Wegwerfhöschen nach Anspruch 1,
**dadurch gekennzeichnet**, daß eine eine Innenfläche des Höschens bildende innere Lage (12) eine elastisch dehnbare, wasserdurchlässige Schicht, eine eine Außenfläche des Höschens bildende äußere Lage (13) eine elastisch dehnbare, wasserundurchlässige Schicht und die Bündchenelemente (6) eine feuchtigkeitsdurchlässige Schicht enthalten.

## Claims

1. Disposable panties with a hip opening (2) having a first elastic element (4), a front part (9) and a back part (10) and side edges arranged on each of these, opposite one another, and with two leg openings provided with ring-like, second elastic elements (5), wherein a cuff element (6) having a third elastic element (7) is arranged in each free end region spaced inside the second elastic element (5) and is lifted on to the skin of the user by the force of contraction of the third elastic element (7), characterized in that, with the panties (6) unfolded and the opposite side edges of a front part (9) and a back part (10) not yet connected, the second elastic elements (5) for the respective leg openings (3) are so arranged that they run inwardly in a curve, while the third elastic elements (7) for the cuff elements (6) a positioned substantially parallel to the longitudinal axis of the panties (1), so that after fitting on the body of the user, elastically stretchable lines formed by the second elastic elements (5) in places intersect elastically stretchable lines formed by the third elastic elements (7).

2. Disposable panties according to claim 1, characterized in that an inner layer (12) forming an inner surface of the panties comprises an elastically stretchable sheet permeable to water, an outer layer (13) forming an outer surface of the panties comprises an elastically stretchable sheet impermeable to water and the cuff element (6) comprises a sheet permeable to moisture.

## Revendications

1. Petite culotte jetable, comportant une ouverture de hanches (2) qui présente un premier élément élastique (4), une partie avant (9) et une partie arrière (10), ainsi que des bords latéraux mutuellement opposés, prévus sur chacune de ces parties, et deux ouvertures de passage des jambes, munies de deuxièmes éléments élastiques (5) du type circulaire, et dans laquelle un petit volant (6), présentant des troisièmes éléments élastiques (7) dans sa zone marginale libre, est disposé à distance à l'intérieur des deuxièmes éléments élastiques (5) et relevé en direction de la peau de l'utilisateur sous la force de contraction des troisièmes éléments élastiques (7), caractérisée en ce que lorsque la petite culotte (1) est étalée alors que les bords latéraux opposés d'une partie avant (9) et d'une partie arrière (10) ne sont pas encore raccordés, les deuxièmes éléments élastiques (5) destinés aux ouvertures de passage des jambes respectives (3) sont agencés de telle manière qu'ils dessinent une courbe vers l'intérieur, tandis que les troisièmes éléments élastiques (7) destinés aux petits volants (6) sont substantiellement parallèles à l'axe longitudinal de la petite culotte (1), de sorte qu'après l'enfilage au corps de l'utilisateur, des lignes élastiques étirables formées par les deuxièmes éléments élastiques (5) coupent partiellement des lignes élastiques étirables formées par les troisièmes éléments élastiques (7).

2. Petite culotte jetable selon la revendication 1, caractérisée en ce qu'une couche intérieure (12) constituant une surface intérieure de la petite culotte contient une couche élastique étirable et perméable à l'eau, en ce qu'une couche extérieure (13) constituant une surface extérieure de la petite culotte contient une couche élastique étirable et imperméable à l'eau, et en ce que les petits volants (6) contiennent une couche perméable aux liquides.
